(19) Europäisches Patentamt
European Patent Office
Office européen des brevets

(11) Veröffentlichungsnummer : **0 281 789 B1**

(12) # EUROPÄISCHE PATENTSCHRIFT

(45) Veröffentlichungstag der Patentschrift : **17.04.91 Patentblatt 91/16**

(51) Int. Cl.$^5$ : **C07D 491/04,** A61K 31/435, // (C07D491/04, 307:00, 221:00)

(21) Anmeldenummer : **88102124.0**

(22) Anmeldetag : **13.02.88**

(54) **Benzylaminoaryl-dihydropyridinelactone, Verfahren zu ihrer Herstellung und ihre Verwendung in Arzneimitteln.**

Die Akte enthält technische Angaben, die nach dem Eingang der Anmeldung eingereicht wurden und die nicht in dieser Patentschrift enthalten sind.

(30) Priorität : **26.02.87 DE 3706204**

(43) Veröffentlichungstag der Anmeldung : **14.09.88 Patentblatt 88/37**

(45) Bekanntmachung des Hinweises auf die Patenterteilung : **17.04.91 Patentblatt 91/16**

(84) Benannte Vertragsstaaten : **AT BE CH DE ES FR GB GR IT LI NL SE**

(56) Entgegenhaltungen :
**EP-A- 0 231 511**
**GB-A- 1 552 911**

(73) Patentinhaber : **BAYER AG
W-5090 Leverkusen 1 Bayerwerk (DE)**

(72) Erfinder : **Goldmann, Siegfried, Dr.
Am Osterholz 91
W-5600 Wuppertal 11 (DE)**
Erfinder : **Gross, Rainer, Prof. Dr.
Platzhofstrasse 23
W-5600 Wuppertal 1 (DE)**
Erfinder : **Bechem, Martin, Dr.
Obere Bergerheide 4
W-5600 Wuppertal 1 (DE)**
Erfinder : **Kayser, Michael, Dr.
Fleyerstrasse 231
W-5800 Hagen (DE)**
Erfinder : **Schramm, Matthias, Dr.
Paffrather Strasse 38
W-5000 Köln 80 (DE)**
Erfinder : **Hebisch, Siegbert, Dr.
Herzogstrasse 129
W-4200 Oberhausen 11 (DE)**

**EP 0 281 789 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft Benzylaminoaryl-dihydropyridinlactone, Verfahren zu ihrer Herstellung sowie ihre Verwendung in Arzneimitteln, insbesondere in kreislaufbeeinflussenden Arzneimitteln.

Die vorliegende Erfindung betrifft Benzylaminoaryl-dihydropyridinlactone der allgemeinen Formel (I)

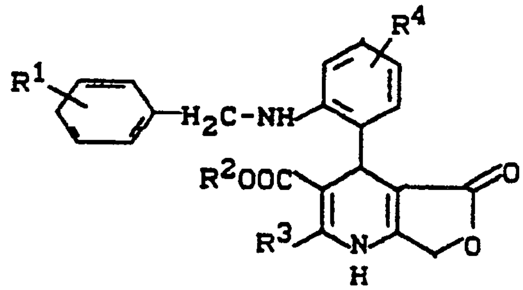

(I)

in welcher

$R^1$ -für Wasserstoff, Halogen, Cyano, Nitro, $C_6$-$C_{12}$-Aryl, $C_1$-$C_8$-Alkyl, $C_1$-$C_8$-Alkoxy, $C_1$-$C_6$-Alkylthio, Trifluormethyl, Trifluormethoxy, Trifluormethylthio, Difluormethoxy, Di-$C_1$-$C_5$-alkylamino, $C_1$-$C_6$-Alkoxycarbonyl oder $C_1$-$C_6$-Alkylsulfonyl steht,

$R^2$ -für Wasserstoff steht, oder

-für einen geradkettigen, verzweigten oder cyclischen, gesättigten oder ungesättigten Kohlenwasserstoffrest mit bis zu 10 Kohlenstoffatomen steht, der gegebenenfalls durch $C_1$-$C_6$-Alkoxy, $C_1$-$C_6$-Alkylthio, $C_1$-$C_6$-Alkylsulfonyl, Halogen, Cyano, Hydroxy, Pyridyl, Thienyl, Pyrimidyl, Piperidinyl, Phenyl oder durch eine Aminogruppe substituiert ist, wobei die Aminogruppe zwei gleiche oder verschiedene Substituenten aus der Reihe $C_1$-$C_5$-Alkyl, Phenyl oder Benzyl trägt,

$R^3$ -für $C_1$-$C_5$-Alkyl steht, oder

-für Cyano, Hydroxymethyl oder Formyl steht und

$R^4$ -für Wasserstoff, Halogen, $C_1$-$C_5$-Alkyl oder Trifluormethyl steht,

in Form ihrer Isomeren, Isomerengemische, Racemate, optischen Antipoden und deren physiologisch unbedenkliche Salze.

Die erfindungsgemäßen Verbindungen können in Form ihrer Salze vorliegen. Im allgemeinen sind dies Salze der erfindungsgemäßen Stoffe mit anorganischen oder organischen Säuren, Bevorzugt sind dies physiologisch unbedenkliche Salze der erfindungsgemäßen Stoffe mit anorganischen oder organischen Säuren. Als Beispiele seien genannt: Hydrohalogenide wie beispielsweise Hydrochloride oder Hydrobromide, oder Hydrogensulfate, Sulfate, Hydrogenphosphate, Formiate, Acetate, Propionate, Maleate, Citrate, Fumarate, Tartrate, Lactate oder Benzoate.

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E.L. Eliel, Stereochemistry of Carbon Compounds, McGraw Hill, 1962).

Bevorzugt seien Verbindungen der allgemeinen Formel (I) genannt, in welcher

$R^1$ -für Wasserstoff, Fluor, Chlor, Brom, Iod, Cyano, Nitro, Phenyl, $C_1$-$C_6$-Alkyl, $C_1$-$C_6$-Alkoxy, $C_1$-$C_4$-Alkylthio, Trifluormethyl, Trifluormethoxy, Difluormethoxy, Di-$C_1$-$C_3$-alkylamino, $C_1$-$C_4$-Alkoxycarbonyl oder $C_1$-$C_4$-Alkylsulfonyl steht,

$R^2$ -für Wasserstoff, oder

-für einen geradkettigen oder verzweigten Kohlenwasserstoffrest mit bis zu 8 Kohlenstoffatomen steht, der durch $C_1$-$C_4$-Alkoxy, $C_1$-$C_4$-Alkylthio, $C_1$-$C_4$-Alkylsulfonyl, Fluor, Chlor, Brom, Iod, Cyano, Hydroxy, Pyridyl, Pyrimidyl, Phenyl oder durch eine Aminogruppe substituiert sein kann, wobei die Aminogruppe zwei gleiche oder verschiedene Substituenten aus der Reihe $C_1$-$C_3$-Alkyl oder Benzyl trägt,

$R^3$ -für $C_1$-$C_3$-Alkyl oder für Cyano steht und

$R^4$ -für Wasserstoff, Fluor, Chlor, Brom, $C_1$-$C_3$-Alkyl oder Trifluormethyl steht,

in Form ihrer Isomeren, Isomerengemische, Racemate, optischen Antipoden und deren physiologisch unbedenkliche Salze.

Besonders bevorzugt seien Verbindungen der allgemeinen Formel (I) genannt, in welcher

$R^1$ -für Wasserstoff, Fluor, Chlor, Brom, Nitro, Phenyl, $C_1$-$C_4$-Alkyl, $C_1$-$C_4$-Alkoxy, Trifluormethyl oder Dimethylamino steht,

$R^2$ -für geradkettiges oder verzweigtes Alkyl mit bis zu 6 Kohlenstoffatomen steht, das durch Methoxy, Fluor, Chlor, Cyano, Hydroxy, Pyridyl, Phenyl oder N-Benzyl-N-methyl-amino substituiert sein kann,

$R^3$ -für Methyl steht und

$R^4$ -für Wasserstoff, Fluor oder Chlor steht,

in Form ihrer Isomeren, Isomerengemische, Racemate, optischen Antipoden und deren physiologisch unbedenkliche Salze.

Die erfindungsgemäßen Verbindungen der allgemeinen Formel (I) erhält man, wenn man Aminoverbindungen der allgemeinen Formel (II)

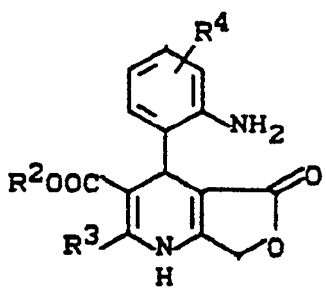

(II),

oder deren Salze in welcher

$R^2$, $R^3$ und $R^4$ die oben angegebene Bedeutung haben, mit Benzylhalogeniden der allgemeinen Formel (III)

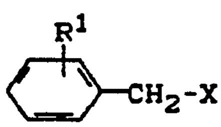

(III),

in welcher

$R^1$ die angegebenen Bedeutung hat und

X -für Halogen, bevorzugt für Chlor oder Brom steht,

gegebenenfalls in Anwesenheit von Basen, gegebenenfalls in Anwesenheit eines inerten Lösemittels umsetzt.

Verwendet man als Ausgangsstoffe 4-(2-Aminophenyl)-2-methyl-5-oxo-1,4,5,7-tetrahydrofuro [3,4-b] pyridin-3-carbonsäure-methylester und Benzylchlorid, so läßt sich die Reaktion durch folgendes Schema verdeutlichen :

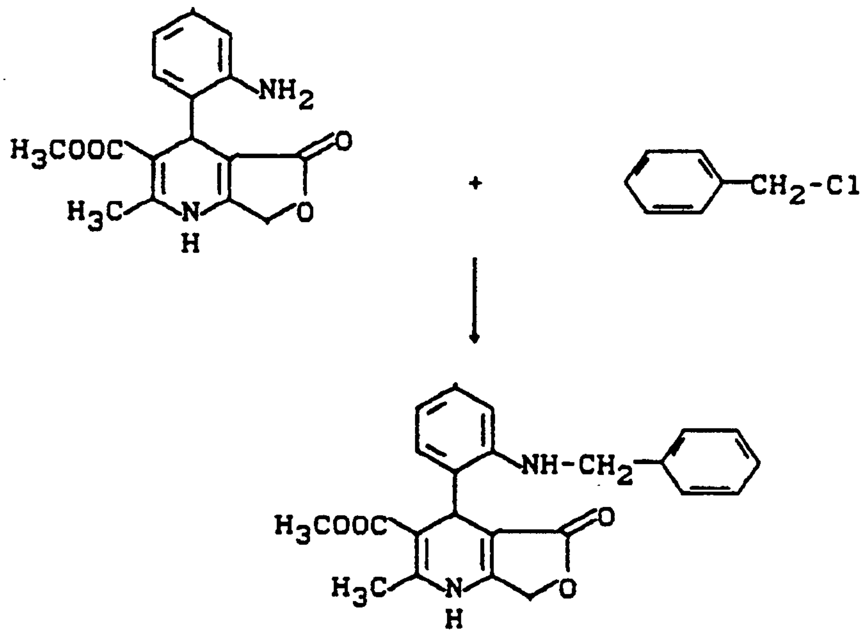

Als Lösemittel eignen sich die üblichen organischen Lösemittel, die sich unter den Reaktionsbedingungen nicht verändern. Hierzu gehören bevorzugt Ether wie Diethylether, Butylmethylether, Dioxan, Tetrahydrofuran oder Glykoldimethylether, oder Halogenkohlenwasserstoffe wie Methylenchlorid, Chloroform, Tetrachlorkohlenstoff oder 1,2-Dichlorethan, oder Kohlenwasserstoffe wie Benzol, Toluol, Xylol, Hexan, oder Erdölfraktionen, oder Amide wie Dimethylformamid, Dimethylacetamid oder Hexamethylphosphorsäuretriamid, oder Essigester, Aceton, Acetonitril, Dimethylsulfoxid oder Pyridin. Ebenso ist es möglich, Gemische der genannten Lösemittel einzusetzen.

Als Basen eignen sich die üblichen anorganischen oder organischen Basen. Hierzu gehören bevorzugt Alkali- oder Erdalkalihydroxide wie Natriumhydroxid, Kaliumhydroxid oder Bariumhydroxid, oder Alkalicarbonate wie Natriumcarbonat, Natriumhydrogencarbonat oder Kaliumcarbonat, oder Alkalialkoholate wie beispielsweise Natriummethanolat, Natriumethanolat, Kaliummethanolat, Kaliumethanolat oder Kalium-tert. butanolat, oder organische Amine wie Trialkylamine beispielsweise Triethylamin oder Ethyldiisopropylamin, oder Basen wie Pyridin, Dimethylaminopyridin, Chinolin, Isochinolin, Methylpiperidin oder Methylmorpholin. Besonders bevorzugt wird Triethylamin oder Kaliumcarbonat eingesetzt.

Die Reaktion kann in einem Temperaturbereich von 0°C bis +100°C, bevorzugt von +10°C bis +50°C durchgeführt werden.

Die Umsetzung kann bei Normaldruck aber auch bei erhöhtem oder bei erniedrigtem Druck durchgeführt werden. Im allgemeinen arbeitet man bei Normaldruck.

Bei der Umsetzung wird das Benzylhalogenid im allgemeinen in einer Menge von 1 bis 3, bevorzugt von 1 bis 1,5 Mol, bezogen auf 1 Mol der Aminoverbindung eingesetzt. Die Base wird im allgemeinen in einer Menge 1 bis 5 Mol, bevorzugt von 1 bis 2,5 Mol, bezogen auf 1 Mol des Benzylhalogenids eingesetzt. Besonders bevorzugt verwendet man molare Mengen aller Reaktanden.

Die als Ausgangsverbindung eingesetzten Aminoverbindungen der allgemeinen Formel (II) bzw. deren Salze sind neu und können hergestellt werden, indem man
Nitroverbindungen der allgemeinen Formel (IV)

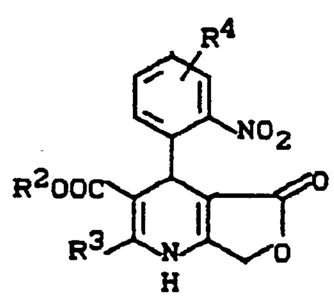

(IV)

in welcher

$R^2$, $R^3$ und $R^4$ die angegebene Bedeutung haben, gegebenenfalls in Anwesenheit eines Katalysators, gegebenenfalls in Anwesenheit einer Säure und gegebenenfalls in Anwesenheit eines inerten Lösemittels in an sich bekannter Weise reduziert.

Verwendet man als Ausgangsstoffe 2-Methyl-4-(2-nitrophenyl)-5-oxo-1,4,5,7-tetrahydrofuro [3,4-b] pyridin-3-carbonsäure-methylester, so läßt sich die Reduktion durch folgendes Schema verdeutlichen :

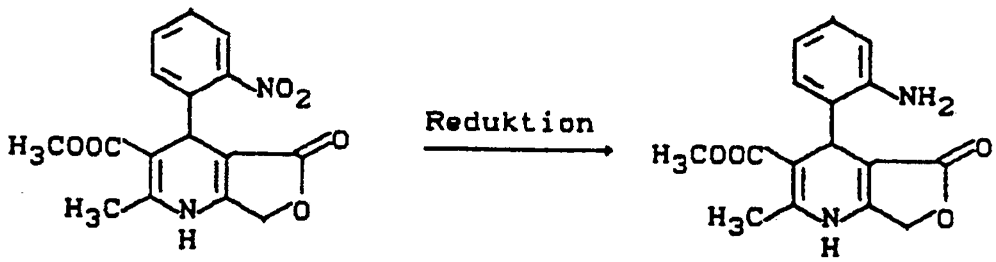


Die Reduktion erfolgt in an sich bekannter Weise, bevorzugt durch Hydrierung mit Metallkatalysatoren wie beispielsweise Platin, Palladium, Palladium auf Tierkohle oder Raney-Nickel in Anwesenheit von Säuren.

Als Säuren können starke Mineralsäuren aber auch organische Säuren eingesetzt werden. Bevorzugt sind Halogenwasserstoffsäuren wie Chlorwasserstoffsäure oder Bromwasserstoffsäure, Schwefelsäure, Phosphorsäure, Perchlorsäure, oder organische Säuren wie Essigsäure, Trifluoressigsäure, Trichloressigsäure, oder Methansulfonsäure, Ethansulfonsäure, Benzolsulfonsäure oder bevorzugt p-Toluolsulfonsäure.

Der Katalysator wird hierbei im allgemeinen in einer Menge von 0,1 bis 50 Mol-%, bevorzugt von 1 bis 10 Mol-%, bezogen auf die Nitroverbindung eingesetzt.

Die Hydrierung erfolgt im allgemeinen in einem Temperaturbereich von –20°C bis +100°C, bevorzugt im Bereich von 0°C bis +50°C.

Im allgemeinen erfolgt die Hydrierung mit einem Überdruck von 5 bis 100 bar, bevorzugt von 10 bis 80 bar Wasserstoffdruck. Es ist ebenso möglich, die Hydrierung bei Normaldruck durchzuführen.

Als Lösemittel für die Hydrierung eignen sich Wasser und/oder inerte organische Lösemittel. Bevorzugt gehören hierzu Alkohole wie Methanol, Ethanol, Propanol oder Isopropanol, oder Ether wie Diethylether, Dioxan, Tetrahydrofuran, Glykolmonomethylether oder Glykoldimethylether, Halogenkohlenwasserstoffe wie Methylenchlorid, Chloroform oder Tetrachlorkohlenstoff, oder Eisessig, Dimethylformamid, Essigester oder Aceton. Ebenso ist es möglich Gemische der genannten Lösemittel einzusetzen.

Besonders bevorzugt wird die Reduktion mit Raney-Nickel in Alkoholen mit Wasserstoffüberdruck durchgeführt.

Die Reduktion kann aber ebenso mit Metallen wie Zink, Zinn oder Eisen in Anwesenheit von Säuren wie Essigsäure oder Salzsäure wie es von R. Schröter in Houben-Weyl's "Methoden der organischen Chemie" VI/1, 363 ff beschrieben wird, durchgeführt werden.

Die als Ausgangsstoffe verwendeten Nitroverbindungen der allgemeinen Formel (IV) sind bekannt oder können nach bekannten Methoden hergestellt werden [DE-OS 32 06 671].

Die erfindungsgemäßen Verbindungen zeigen ein nicht vorhersehbares, wertvolles pharmakologisches Wirkspektrum. Sie beeinflussen die Kontraktionskraft des Herzens und die Tonus der glatten Muskulatur. Sie können deshalb in Arzneimitteln zur Beeinflussung des pathlogisch veränderten Blutdrucks, als Koronartherapeutika und zur Behandlung der Herzinsuffizienz, eingesetzt werden. Darüber hinaus können sie zur Behandlung von Herzrhythmusstörungen, zur Senkung des Blutzuckers, zur Abschwellung von Schleimhäuten und zur Beeinflussung des Salz- und Flüssigkeitshaushaltes verwendet werden.

Die Herzwirkung wurde an isolierten Vorhöfen von Meerschweinschenherzen gefunden.

Dazu werden die linken Vorhöfe von Meerschweinchenherzen isoliert, und in ein auf 32°C thermostatisiertes Organbad gehängt. Als Inkubationsmedium dient eine Krebs-Henselei-Lösung mit folgender Zusammensetzung (118,5 mmol/l NaCl, 4,75 mmol/l KCl, 1,19 mmol/l $KH_2PO_4$, 119 mmol/l $MgSO_4$, 25 mmol/l $NaHCO_3$, 0,013 mmol/l NaEDTA, 1,8 mmol/l $CaCl_2$) unter Zusatz von 10 mmol/l Glukose als energieliefemdes Substrat. Die Lösung wird mit Carbogen (95% $CO_2$, 5% $O_2$) zur Aufrechterhaltung eines pH-Wertes von 7,4 begast. Die linken Vorhöfe werden unter Einstellung eines bestimmten Grundtonus in das Organbad eingespannt, und die Spannung mittels eines Kraftaufnehmers registriert. Unter periodischer elektrischer Reizung werden die dabei folgenden Kontraktionen fortlaufend auf einem Schnellschreiber aufgezeichnet. In Gegenwart der jeweiligen erfindungsgemäßen Verbindungen kommt es dabei zu einer prozentualen Veränderung gegenüber dem gleich

100% gesetzten Ausgangswert:

| Beispiel | Konzentration (g/l) | %-Änderung der Kontraktionskraft |
|---|---|---|
| 4 | $10^{-3}$ | +57 |
| 5 | $10^{-3}$ | +33 |
| 6 | $10^{-3}$ | +109 |
| 7 | $10^{-3}$ | +120 |
| 8 | $10^{-3}$ | +54 |
| 9 | $10^{-3}$ | +56 |
| 10 | $10^{-3}$ | +80 |
| 12 | $10^{-3}$ | +70 |

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche Öle (z.B. Erdnuß/Sesamöl), Alkohole (z.b : Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate, Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verarbreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden

muß. Im Falle der Applikation größerer Mengen kann es empfehlenswert sein, diese in mehreren Einzelgaben über den Tag zu verteilen.

Herstellungsbeispiele

Beispiel 1

4-(2-Aminophenyl)-2-methyl-5-oxo-1,4,5,7-tetrahydrofuro [3,4-b] pyridin-3-carbonsäure-ethylester Hydrochlorid

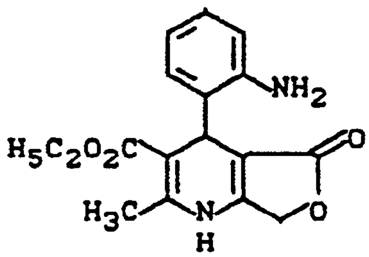

x HCl

58 mmol4-(2-Nitrophenyl)-2-methyl-5-oxo-1,4,5,7-tetrahydrofuro [3,4-b] pyridin-3-carbonsäureethylester werden in 200 ml Tetrahydrofuran gelöst und mit 2 g Raney-Nickel versetzt. Bei 50 bar Wasserstoffdruck wird 1,5 h hydriert. Die Lösung wird eingedampft, mit verdünnter Salzsäure versetzt, abgesaugt und der Rückstand getrocknet.
Ausbeute : 56% der Theorie
Schmp. : 175-183°C

Analog Beispiel 1 wurden hergestellt :

Beispiel 2

4-(2-Aminophenyl)-2-methyl-5-oxo-1,4,5,7-tetrahydrofuro [3,4-b] pyridin-3-carbonsäure-methylester

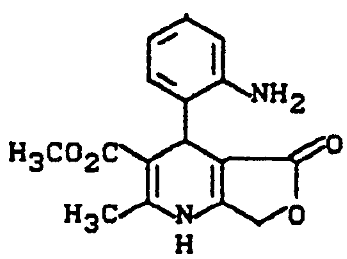

Ausbeute : 80% der Theorie
Schmp. : 193-195°C

Beispiel 3

4-(2-Aminophenyl)-2-methyl-5-oxo-1,4,5,7-tetrahydrofuro [3,4-b] pyridin-3-carbonsäure-butylester

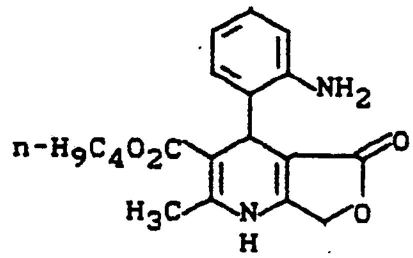

Ausbeute : 60% der Theorie
Schmp. : 167-169°C

Beispiel 4

4-(2-Benzylaminophenyl)-2-methyl-5-oxo-1,4,5,7-tetrahydrofuro [3,4-b] pyridin-3-carbonsäure-ethylester

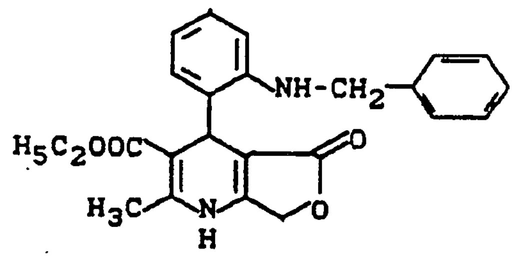

3,5 g (10 mmol) 4-(2-Aminophenyl)-2-methyl-5-oxo-1,4,5,7-tetrahydrofuro [3,4-b] pyridin-3-carbonsäure-ethylester-Hydrochlorid und 1,4 ml (11 mmol) Benzylbromid werden in 50 ml absolutem Aceton gelöst, mit 1,4 g Kaliumcarbonat versetzt und über Nacht bei Raumtemperatur gerührt. Es wird eingeengt, mit Wasser versetzt, abgesaugt und aus Methanol umkristallisiert.
Ausbeute : 67% der Theorie
Schmp. : 180-183°C

Analog Beispiel 4 wurden hergestellt :

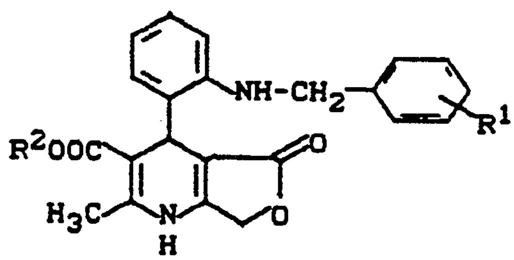

| Bsp.-Nr. | $R^2$ | $R^1$ | Schmp. [°C] | Ausbeute [% d. Th.] |
|---|---|---|---|---|
| 5 | $-C_2H_5$ | 3-Cl | 165-167 | 55 % |

| Bsp.-Nr. | $R^2$ | $R^1$ | Schmp. [°C] | Ausbeute [% d. Th.] |
|---|---|---|---|---|
| 6 | $-C_2H_5$ | $3-H_3C-$ | 166-177 | 52% |
| 7 | $-C_2H_5$ | 2-Cl- | 210-217 | 57% |
| 8 | $-C_2H_5$ | $4-H_3C-$ | 166-169 | 50% |
| 9 | $-C_2H_5$ | $4-NO_2-$ | 204-206 | 45% |
| 10 | $-CH_3$ | $4-H_3C-$ | 214 | 40% |
| 11 | $-C_2H_5$ | $4-C_6H_5$ | 208 | 77% |
| 12 | $-CH_3$ | H | 213 | 40% |

**Ansprüche**

**Patentansprüche für die Vertragsstaaten : AT, BE, CH, DE, FR, GB, IT, LI, NL, SE**

1. Benzylaminoaryl-dihydropyridinlactone der allgemeinen Formel (I)

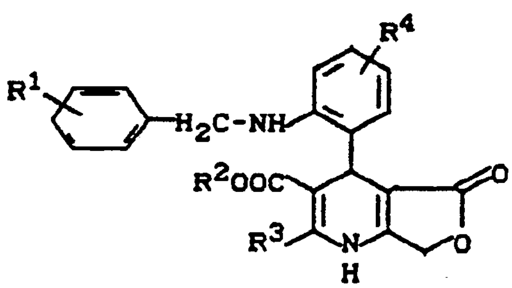

(I)

in welcher

$R^1$ -für Wasserstoff, Halogen, Cyano, Nitro, $C_6$-$C_{12}$-Aryl, $C_1$-$C_8$-Alkyl, $C_1$-$C_8$-Alkoxy, $C_1$-$C_6$-Alkylthio, Trifluormethyl, Trifluormethoxy, Trifluormethylthio, Difluormethoxy, Di-$C_1$-$C_5$-alkylamino, $C_1$-$C_6$-Alkoxycarbonyl oder $C_1$-$C_6$-Alkylsulfonyl steht,

$R^2$ -für Wasserstoff steht, oder

-für einen geradkettigen, verzweigten oder cyclischen, gesättigten oder ungesättigten Kohlenwasserstoffrest mit bis zu 10 Kohlenstoffatomen steht, der gegebenenfalls durch $C_1$-$C_6$-Alkoxy, $C_1$-$C_6$-Alkylthio, $C_1$-$C_6$-Alkylsulfonyl, Halogen, Cyano, Hydroxy, Pyridyl, Thienyl, Pyrimidyl, Piperidinyl, Phenyl oder durch eine Aminogruppe substituiert ist, wobei die Aminogruppe zwei gleiche oder verschiedene Substituenten aus der Reihe $C_1$-$C_5$-Alkyl, Phenyl oder Benzyl trägt,

$R^3$ -für $C_1$-$C_5$-Alkyl steht, oder

-für Cyano, Hydroxymethyl oder Formyl steht und

$R^4$ -für Wasserstoff, Halogen, $C_1$-$C_5$-Alkyl oder Trifluormethyl steht,

in Form ihrer Isomeren, Isomerengemische, Racemate, optischen Antipoden und deren physiologisch unbedenkliche Salze.

2. Verbindungen gemäß Formel I in Anspruch 1, in welcher

$R^1$ -für Wasserstoff, Fluor, Chlor, Brom, Iod, Cyano, Nitro, Phenyl, $C_1$-$C_6$-Alkyl, $C_1$-$C_6$-Alkoxy, $C_1$-$C_4$-Alkylt-

hio, Trifluormethyl, Trifluormethoxy, Difluormethoxy, Di-$C_1$-$C_3$-alkylamino, $C_1$-$C_4$-Alkoxycarbonyl oder $C_1$-$C_4$-Alkylsulfonyl steht,

$R^2$ -für Wasserstoff, oder

-für einen geradkettigen oder verzweigten Kohlenwasserstoffrest mit bis zu 8 Kohlenstoffatomen steht, der durch $C_1$-$C_4$-Alkoxy, $C_1$-$C_4$-Alkylthio, $C_1$-$C_4$-Alkylsulfonyl, Fluor, Chlor, Brom, Iod, Cyano, Hydroxy, Pyridyl, Pyrimidyl, Phenyl oder durch eine Aminogruppe substituiert sein kann, wobei die Aminogruppe zwei gleiche oder verschiedene Substituenten aus der Reihe $C_1$-$C_3$-Alkyl oder Benzyl trägt,

$R^3$ -für $C_1$-$C_3$-Alkyl oder für Cyano steht und

$R^4$ -für Wasserstoff, Fluor, Chlor, Brom, $C_1$-$C_3$-Alkyl oder Trifluormethyl steht,

in Form ihrer Isomeren, Isomerengemische, Racemate, optischen Antipoden und deren physiologisch unbedenkliche Salze.

3. Verbindungen gemäß Formel I in Anspruch 1, in welcher

$R^1$ -für Wasserstoff, Fluor, Chlor, Brom, Nitro, Phenyl, $C_1$-$C_4$-Alkyl, $C_1$-$C_4$-Alkoxy, Trifluormethyl oder Dimethylamino steht,

$R^2$ -für geradkettiges oder verzweigtes Alkyl mit bis zu 6 Kohlenstoffatomen steht, das durch Methoxy, Fluor, Chlor, Cyano, Hydroxy, Pyridyl, Phenyl oder N-Benzyl-N-methyl-amino substituiert sein kann,

$R^3$ -für Methyl steht und

$R^4$ -für Wasserstoff, Fluor oder Chlor steht,

in Form ihrer Isomeren, Isomerengemische, Racemate, optischen Antipoden und deren physiologisch unbedenkliche Salze.

4. Verbindungen gemäß Ansprüche 1-3 zur Bekämpfung von Erkrankungen.

5. Arzneimittel enthaltend als Wirkstoff einer Verbindung gemäß Ansprüche 1-3.

6. Verfahren zur Herstellung von Benzylaminoaryldihydropyridinlactonen der allgemeinen Formel (I)

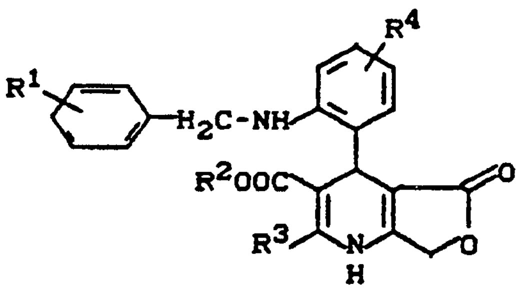

(I)

in welcher

$R^1$ -für Wasserstoff, Halogen, Cyano, Nitro, $C_6$-$C_{12}$-Aryl, $C_1$-$C_8$-Alkyl, $C_1$-$C_8$-Alkoxy, $C_1$-$C_6$-Alkylthio, Trifluormethyl, Trifluormethoxy, Trifluormethylthio, Difluormethoxy, Di-$C_1$-$C_5$-alkylamino, $C_1$-$C_6$-Alkoxycarbonyl oder $C_1$-$C_6$-Alkylsulfonyl steht,

$R^2$ -für Wasserstoff steht, oder

-für einen geradkettigen, verzweigten oder cyclischen, gesättigten oder ungesättigten Kohlenwasserstoffrest mit bis zu 10 Kohlenstoffatomen steht, der gegebenenfalls durch $C_1$-$C_6$-Alkoxy, $C_1$-$C_6$-Alkylthio, $C_1$-$C_6$-Alkylsulfonyl, Halogen, Cyano, Hydroxy, Pyridyl, Thienyl, Pyrimidyl, Piperidinyl, Phenyl oder durch eine Aminogruppe substituiert ist, wobei die Aminogruppe zwei gleiche oder verschiedene Substituenten aus der Reihe $C_1$-$C_5$-Alkyl, Phenyl oder Benzyl trägt,

$R^3$ -für $C_1$-$C_5$-Alkyl steht, oder

-für Cyano, Hydroxymethyl oder Formyl steht und

$R^4$ -für Wasserstoff, Halogen, $C_1$-$C_5$-Alkyl oder Trifluormethyl steht,

in Form ihrer Isomeren, Isomerengemische, Racemate, optischen Antipoden und deren physiologisch unbedenkliche Salze, dadurch gekennzeichnet, daß man Aminoverbindungen der allgemeinen Formel (II)

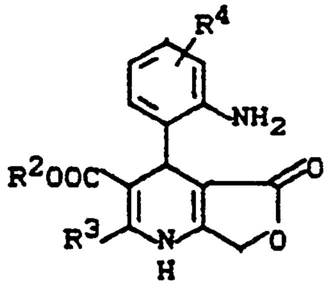

(II),

oder deren Salze in welcher

$R^2$, $R^3$ und $R^6$ die oben angegebene Bedeutung haben, mit Benzylhalogeniden der allgemeinen Formel (III)

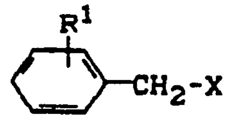

(III),

in welcher

$R^1$ die angegebene Bedeutung hat und

X-für Halogen, bevorzugt für Chlor oder Brom steht, gegebenenfalls in Anwesenheit von Basen und/oder gegebenenfalls in Anwesenheit eines inerten Lösemittels umsetzt.

7. Verfahren gemäß Anspruch 6 zur Herstellung von Verbindungen der Formel I, in der

$R^1$ -für Wasserstoff, Fluor, Chlor, Brom, Iod, Cyano, Nitro, Phenyl, $C_1$-$C_8$-Alkyl, $C_1$-$C_6$-Alkoxy, $C_1$-$C_4$-Alkylthio, Trifluormethyl, Trifluormethoxy, Difluormethoxy, Di-$C_1$-$C_3$-alkylamino, $C_1$-$C_4$-Alkoxycarbonyl oder $C_1$-$C_4$-Alkylsulfonyl steht,

$R^2$ -für Wasserstoff steht, oder

-für einen geradkettigen, verzweigten oder cyclischen gesättigten oder ungesättigten Kohlenwasserstoffrest mit bis zu 10 Kohlenstoffatomen steht, der gegebenenfalls durch $C_1$-$C_6$-Alkoxy, $C_1$-$C_6$-Alkylthio, $C_1$-$C_6$-Alkylsulfonyl, Halogen, Cyano, Hydroxy, Pyridyl, Thienyl, Pyrimidyl, Piperidinyl, Phenyl oder durch eine Aminogruppe substituiert ist, wobei die Aminogruppe zwei gleiche oder verschiedene Substituenten aus der Reihe $C_1$-$C_5$-Alkyl, Phenyl oder Benzyl trägt,

$R^3$ -für $C_1$-$C_5$-Alkyl steht, oder

-für Cyano, Hydroxymethyl oder Formyl steht und

$R^4$ -für Wasserstoff, Halogen, $C_1$-$C_5$-Alkyl oder Trifluormethyl steht,

in Form ihrer Isomeren, Isomerengemische, Racemate, optischen Antipoden

und deren physiologisch unbedenkliche Salze.

8. Verfahren gemäß Anspruch 6 zur Herstellung von Verbindungen der Formel I, in der

$R^1$ -für Wasserstoff, Fluor, Chlor, Brom, Nitro, Phenyl, $C_1$-$C_4$-Alkyl, $C_1$-$C_4$-Alkoxy, Trifluormethyl oder Dimethylamino steht,

$R^2$ -für geradkettiges oder verzweigtes Alkyl mit bis zu 6 Kohlenstoffatomen steht, das durch Methoxy, Fluor, Chlor, Cyano, Hydroxy, Pyridyl, Phenyl oder N-Benzyl-N-methyl-amino substituiert sein kann,

$R^3$ -für Methyl steht und

$R^4$ -für Wasserstoff, Fluor oder Chlor steht,

in Form ihrer Isomeren, Isomerengemische, Racemate, optischen Antipoden

und deren physiologisch unbedenkliche Salze.

9. Verfahren zur Herstellung von Arzneimitteln gemäß Anspruch 5, dadurch gekennzeichnet, daß man in üblicher Weise die Wirkstoffe gemäß Ansprüche 1-3 mit üblichen Hilfs- und/oder Trägerstoffen vermischt.

10. Verwendung der Verbindungen gemäß Ansprüche 1-3 zur Herstellung von Arzneimitteln zur Koronartherapie, zur Beeinflussung des pathologisch veränderten Blutdrucks, zur Behandlung der Herzinsuffizienz, zur Behandlung von Herzrhythmusstörungen, zur Senkung des Blutzuckers, zur Abschwellung von Schleimhäuten und zur Beeinflussung des Salz- und Flüssigkeitshaushaltes.

**Patentansprüche für die Vertragsstaaten : ES, GR**

1. Verfahren zur Herstellung von Benzylaminoaryldihydropyridinlactonen der allgemeinen Formel (I)

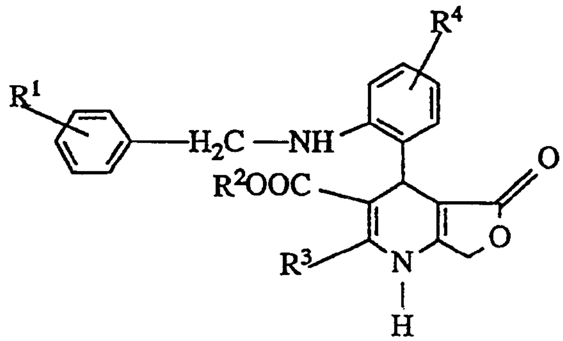

(I)

in welcher

$R^1$ für Wasserstoff, Halogen, Cyano, Nitro, $C_6$-$C_{12}$-Aryl, $C_1$ -$C_8$-Alkyl, $C_1$-$C_8$ Alkoxy, $C_1$-$C_6$-Alkylthio, Trifluormethyl, Trifluormethoxy, Trifluormethylthio, Difluormethoxy, Di-$C_1$-$C_5$-alkylamino, $C_1$-$C_6$-Alkoxycarbonyl oder $C_1$-$C_8$-Alkylsulfonyl steht,

$R^2$ für Wasserstoff steht, oder für einen geradkettigen, verzweigten oder cyclischen, gesättigten oder ungesättigten Kohlenwasserstoffrest mit bis zu 10 Kohlenstoffatomen steht, der gegebenenfalls durch $C_1$-$C_6$-Alkoxy, $C_1$-$C_6$-Alkylthio, $C_1$-$C_6$-Alkylsulfonyl, Halogen, Cyano, Hydroxy, Pyridyl, Thienyl, Pyrimidyl, Piperidinyl, Phenyl oder durch eine Aminogruppe substituiert ist, wobei die Aminogruppe zwei gleiche oder verschiedene Substituenten aus der Reihe $C_1$-$C_5$-Alkyl, Phenyl oder Benzyl trägt,

$R^3$ für $C_1$-$C_5$-Alkyl steht oder für Cyano, Hydroxymethyl oder Formyl steht, und

$R^4$ für Wasserstoff, Halogen, $C_1$-$C_5$-Alkyl oder Trifluormethyl steht,

in Form ihrer Isomeren, Isomerengemische, Racemate, optischen Antipoden und deren physiologisch unbedenkliche Salze, dadurch gekennzeichnet, daß man Aminoverbindungen der allgemeinen Formel (II)

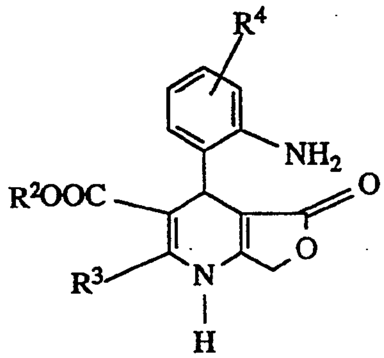

(II)

oder deren Salze, in welcher

$R^2$, $R^3$ und $R^4$ die oben angegebene Bedeutung haben, mit Benzylhalogeniden der allgemeinen Formel (III)

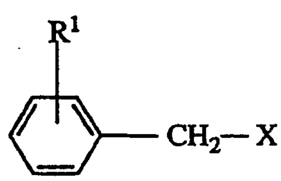

(III)

in welcher

$R^1$ die angegebene Bedeutung hat, und

X für Halogen, bevorzugt für Chlor oder Brom steht,

gegebenenfalls in Anwesenheit von Basen und/oder gegebenenfalls in Anwesenheit eines inerten Lösemittels

umsetzt.

2. Verfahren gemäß Anspruch 1 zur Herstellung von Verbindungen der Formel I, in der

$R^1$ -für Wasserstoff, fluor, Chlor, Brom, Iod, Cyano, Nitro, Phenyl, $C_1$-$C_6$-Alkyl, $C_1$-$C_6$-Alkoxy, $C_1$-$C_4$-Alkylthio, Trifluormethyl, Trifluormethoxy, Difluormethoxy, Di-$C_1$-$C_3$-alkylamino, $C_1$-$C_4$-Alkoxycarbonyl oder $C_1$-$C_4$-Alkylsulfonyl steht,

$R^2$ -für Wasserstoff steht, oder

-für einen geradkettigen, verzweigten oder cyclischen, gesättigten oder ungesättigten Kohlenwasserstoffrest mit bis zu 10 Kohlenstoffatomen steht, der gegebenenfalls durch $C_1$-$C_6$-Alkoxy, $C_1$-$C_6$-Alkylthio, $C_1$-$C_6$-Alkylsulfonyl, Halogen, Cyano, Hydroxy, Pyridyl, Thienyl, Pyrimidyl, Piperidinyl, Phenyl oder durch eine Aminogruppe substituiert ist, wobei die Aminogruppe zwei gleiche oder verschiedene Substituenten aus der Reihe $C_1$-$C_5$-Alkyl, Phenyl oder Benzyl trägt,

$R^3$ -für $C_1$-$C_5$-Alkyl steht, oder

-für Cyano, Hydroxymethyl oder formyl steht und

$R^4$ -für Wasserstoff, Halogen, $C_1$-$C_5$-Alkyl oder Trifluormethyl steht,

in Form ihrer Isomeren, Isomerengemische, Racemate, optischen Antipoden und deren physiologisch unbedenkliche Salze.

3. Verfahren gemäß Anspruch 1 zur Herstellung von Verbindungen der Formel I, in der

$R^1$ -für Wasserstoff, fluor, Chlor, Brom, Nitro, Phenyl, $C_1$-$C_4$-Alkyl, $C_1$-$C_4$-Alkoxy, Trifluormethyl oder Dimethylamino steht,

$R^2$ -für geradkettiges oder verzweigtes Alkyl mit bis zu 6 Kohlenstoffatomen steht, das durch Methoxy, Fluor, Chlor, Cyano, Hydroxy, Pyridyl, Phenyl oder N-Benzyl-N-methyl-amino substituiert sein kann,

$R^3$ -für Methyl steht und

$R^4$ -für Wasserstoff, Fluor oder Chlor steht,

in Form ihrer Isomeren, Isomerengemische, Racemate, optischen Antipoden und deren physiologisch unbedenkliche Salze.

4. Verfahren zur Herstellung von Arzneimitteln, dadurch gekennzeichnet, daß man gemaß Anspruch 1 hergestellte Verbindungen gegebenenfalls mit üblichen Hilfs- und Trägerstoffen in eine geeignete Applikationsform überführt.

5. Verfahren gemaß Anspruch 4 zur Herstellung von Arzneimitteln zur Koronartherapie, zur Beeinflussung des pathologisch veränderten Blutdrucks, zur Behandlung der Herzinsuffizienz, zur Behandlung von Herzrhythmusstörungen, zur Senkung des Blutzuckers, zur Abschwellung von Schleimhäuten und zur Beeinflussung des Salz- und Flüssigkeitshaushaltes.

## Claims

**Claims for the Contracting States : AT, BE, CH, DE, FR, GB, IT, LI, NL, SE**

1. Benzylaminoaryl-dihydropyridinelactones of the general formula (I)

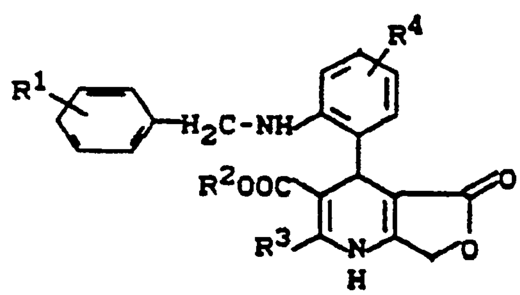

(I)

in which

$R^1$ represents hydrogen, halogen, cyano, nitro, $C_6$-$C_{12}$-aryl, $C_1$-$C_8$-alkyl, $C_1$-$C_8$-alkoxy, $C_1$-$C_6$-alkylthio, trifluoromethyl, trifluoromethoxy, trifluoromethylthio, difluoromethoxy, di-$C_1$-$C_5$-alkylamino, $C_1$-$C_6$-alkoxycarbonyl or $C_1$-$C_6$ -alkylsulphonyl,

$R^2$ represents hydrogen, or represents a straight-chain, branched or cyclic, saturated or unsaturated hydrocarbon radical having up to 10 carbon atoms which is optionally substituted by $C_1$-$C_6$-alkoxy, $C_1$-$C_6$-alkylthio, $C_1$-$C_6$-alkylsulphonyl, halogen, cyano, hydroxyl, pyridyl, thienyl, pyrimidyl, piperidinyl, phenyl or an amino

group, where the amino group carries two identical or different substituents from the series comprising $C_1$-$C_5$-alkyl, phenyl or benzyl, $R^3$ represents $C_1$-$C_5$-alkyl, or represents cyano, hydroxymethyl or formyl, and

$R^4$ represents hydrogen, halogen, $C_1$-$C_5$-alkyl or trifluoromethyl,

in the form of their isomers, isomeric mixtures, racemates or optical antipodes, and their physiologically acceptable salts.

2. Compounds according to formula I in Claim 1, in which

$R^1$ represents hydrogen, fluorine, chlorine, bromine, iodine, cyano, nitro, phenyl, $C_1$-$C_6$-alkyl, $C_1$-$C_6$-alkoxy, $C_1$-$C_4$-alkylthio, trifluoromethyl, trifluoromethoxy, difluoromethoxy, di-$C_1$-$C_3$-alkylamino, $C_1$-$C_4$-alkoxycarbonyl or $C_1$-$C_4$-alkylsulphonyl,

$R^2$ represents hydrogen, or represents a straight-chain or branched hydrocarbon radical which has up to 8 carbon atoms and which may be substituted by $C_1$-$C_4$-alkoxy, $C_1$-$C_4$-alkylthio, $C_1$-$C_4$-alkylsulphonyl, fluorine, chlorine, bromine, iodine, cyano, hydroxyl, pyridyl, pyridmidyl, phenyl or by an amino group, where the amino group carries two identical or different substituents from the series comprising $C_1$-$C_3$-alkyl or benzyl,

$R^3$ represents $C_1$-$C_3$-alkyl or cyano, and

$R^4$ represents hydrogen, fluorine, chlorine, bromine, $C_1$-$C_3$-alkyl or trifluoromethyl,

in the form of their isomers, isomeric mixtures, racemates or optical antipodes, and their physiologically acceptable salts.

3. Compounds according to formula I in Claim 1, in which

$R^1$ represents hydrogen, fluorine, chlorine, bromine, nitro, phenyl, $C_1$-$C_4$-alkyl, $C_1$-$C_4$-alkoxy, trifluoromethyl or dimethylamino,

$R^2$ represents straight-chain or branched alkyl which has up to 6 carbon atoms and which may be substituted by methoxy, fluorine, chlorine, cyano, hydroxyl, pyridyl, phenyl or N-benzyl-N-methylamino

$R^3$ represents methyl, and

$R^4$ represents hydrogen, fluorine or chlorine,

in the form of their isomers, isomeric mixtures, racemates or optical antipodes, and their physiologically acceptable salts.

4. Compounds according to Claims 1-3 for combating disorders.

5. Medicaments containing a compound according to Claims 1-3 as active compound.

6. Process for the preparation of benzylaminoaryldihydropyridinelactones of the general formula (I)

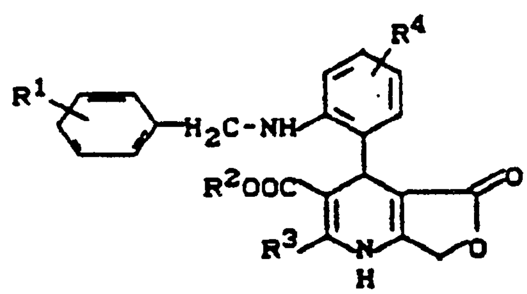

(I)

in which

$R^1$ represents hydrogen, halogen, cyano, nitro, $C_6$-$C_{12}$-aryl, $C_1$-$C_8$-alkyl, $C_1$-$C_8$-alkoxy, $C_1$-$C_6$-alkylthio, trifluoromethyl, trifluoromethoxy, trifluoromethylthio, difluoromethoxy, di-$C_1$-$C_5$-alkylamino, $C_1$-$C_6$-alkoxycarbonyl or $C_1$-$C_6$-alkylsulphonyl,

$R^2$ represents hydrogen, or represents a straight-chain, branched or cyclic, saturated or unsaturated hydrocarbon radical having up to 10 carbon atoms which is optionally substituted by $C_1$-$C_6$-alkoxy, $C_1$-$C_6$-alkylthio, $C_1$-$C_6$-alkylsulphonyl, halogen, cyano, hydroxyl, pyridyl, thienyl, pyrimidyl, piperidinyl, phenyl or an amino group, where the amino group carries two identical or different substituents from the series comprising $C_1$-$C_5$-alkyl, phenyl or benzyl,

$R^3$ represents $C_1$-$C_5$-alkyl, or represents cyano, hydroxymethyl or formyl, and

$R^4$ represents hydrogen, halogen, $C_1$-$C_5$-alkyl or trifluoromethyl,

in the form of their isomers, isomeric mixtures, racemates or optical antipodes, and their physiologically acceptable salts, characterized in that amino compounds of the general formula (II)

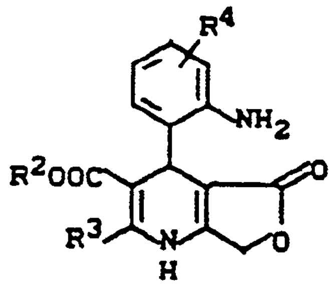

(II)

or their salts in which

$R^2$, $R^3$ and $R^4$ have the abovementioned meaning, are reacted with benzyl halides of the general formula (III)

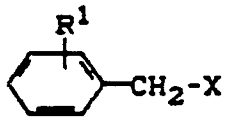

(III)

in which

$R^1$ has the abovementioned meaning, and

X represents halogen, preferably chlorine or bromine,

if appropriate in the presence of bases and/or if appropriate in the presence of an inert solvent.

7. Process according to Claim 6 for the preparation of compounds of the formula I, in which

$R^1$ represents hydrogen, fluorine, chlorine, bromine, iodine, cyano, nitro, phenyl, $C_1$-$C_6$-alkyl, $C_1$-$C_6$-alkoxy, $C_1$-$C_4$-alkylthio, trifluoromethyl, trifluoromethoxy, difluoromethoxy, di-$C_1$-$C_3$-alkylamino, $C_1$-$C_4$-alkoxycarbonyl or $C_1$-$C_4$-alkylsulphonyl,

$R^2$ represents hydrogen, or represents a straight-chain, branched or cyclic, saturated or unsaturated hydrocarbon radical having up to 10 carbon atoms which is optionally substituted by $C_1$-$C_6$-alkoxy, $C_1$-$C_6$-alkylthio, $C_1$-$C_6$-alkylsulphonyl, halogen, cyano, hydroxyl, pyridyl, thienyl, pyrimidyl, piperidinyl, phenyl or an amino group, where the amino group carries two identical or different substituents from the series comprising $C_1$-$C_5$-alkyl, phenyl or benzyl,

$R^3$ represents $C_1$-$C_5$-alkyl, or represents cyano, hydroxymethyl or formyl, and

$R^4$ represents hydrogen, halogen, $C_1$-$C_5$-alkyl or trifluoromethyl,

in the form of their isomers, isomeric mixtures, racemates or optical antipodes, and their physiologically acceptable salts.

8. Process according to Claim 6 for the preparation of compounds of the formula I in which

$R^1$ represents hydrogen, fluorine, chlorine, bromine, nitro, phenyl, $C_1$-$C_4$-alkyl, $C_1$-$C_4$-alkoxy, trifluromethyl or dimethylamino,

$R^2$ represents straight-chain or branched alkyl which has up to 6 carbon atoms and which may be substituted by methoxy, fluorine, chlorine, cyano, hydroxyl, pyridyl, phenyl or N-benzyl-N-methylamino,

$R^3$ represents methyl, and

$R^4$ represents hydrogen, fluorine or chlorine, in the form of their isomers, isomeric mixtures, racemates or optical antipodes, and their physiologically acceptable salts.

9. Process for the preparation of medicaments according to Claim 5, characterized in that the active compounds according to Claims 1-3 are mixed with conventional auxiliaries and/or excipients in a conventional fashion.

10. Use of the compounds according to Claims 1-3 for the preparation of medicaments for coronary therapy, for influencing pathologically altered blood pressure, for treatment of heart insufficiency, for treatment of heart rhythm disturbances, for lowering blood sugar levels, for shrinking mucous membranes and for influencing the salt and liquid balance.

**Claims for the Contracting States ES, GR**

1. Process for the preparation of benzylaminoaryldihydropyridinelactones of the general formula (I)

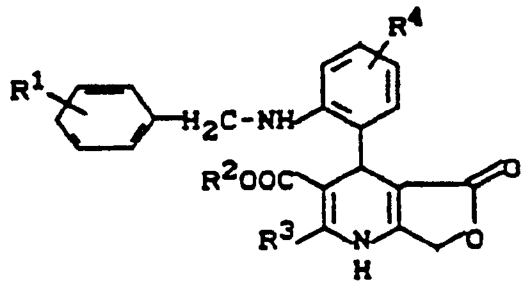

(I)

in which

$R^1$ represents hydrogen, halogen, cyano, nitro, $C_6$-$C_{12}$-aryl, $C_1$-$C_8$-alkyl, $C_1$-$C_8$-alkoxy, $C_1$-$C_6$-alkylthio, trifluoromethyl, trifluoromethoxy, trifluoromethylthio, difluoromethoxy, di-$C_1$-$C_5$-alkylamino, $C_1$-$C_6$-alkoxycarbonyl or $C_1$-$C_8$-alkylsulphonyl,

$R^2$ represents hydrogen, or represents a straightchain, branched or cyclic, saturated or unsaturated hydrocarbon radical having up to 10 carbon atoms which is optionally substituted by $C_1$-$C_6$ -alkoxy, $C_1$-$C_6$-alkylthio, $C_1$-$C_6$-alkylsulphonyl, halogen, cyano, hydroxyl, pyridyl, thienyl, pyrimidyl, piperidinyl, phenyl or an amino group, where the amino group carries two identical or different substituents from the series comprising $C_1$-$C_5$-alkyl, phenyl or benzyl, $R^3$ represents $C_1$-$C_5$-alkyl, or represents cyano, hydroxymethyl or formyl, and

$R^4$ represents hydrogen, halogen, $C_1$-$C_5$-alkyl or trifluoromethyl,

in the form of their isomers, isomeric mixtures, racemates or optical antipodes, and their physiologically acceptable salts, characterized in that amino compounds of the general formula (II)

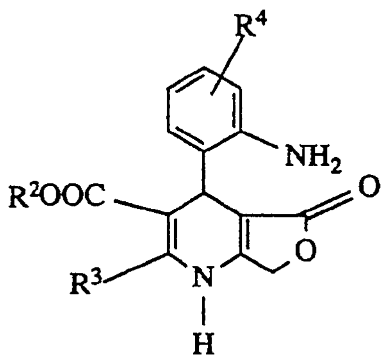

(II)

or their salts in which

$R^2$, $R^3$ and $R^4$ have the abovementioned meaning, are reacted with benzyl halides of the general formula (III)

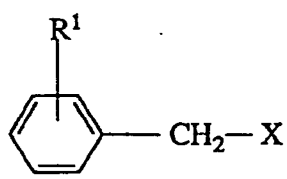

(III)

in which

$R^1$ has the abovementioned meaning, and

X represents halogen, preferably chlorine or bromine,

if appropriate in the presence of bases and/or if appropriate in the presence of an inert solvent.

2. Process according to Claim 1 for the preparation of compounds of the formula I, in which

$R^1$ represents hydrogen, fluorine, chlorine, bromine, iodine, cyano, nitro, phenyl, $C_1$-$C_6$-alkyl, $C_1$-$C_6$-alkoxy, $C_1$-$C_4$-alkylthio, trifluoromethyl, trifluoromethoxy, difluoromethoxy, di-$C_1$-$C_3$-alkylamino, $C_1$-$C_4$-alkoxycarbonyl or $C_1$-$C_4$-alkylsulphonyl,

$R^2$ represents hydrogen, or represents a straight-chain, branched or cyclic, saturated or unsaturated hydrocarbon radical having up to 10 carbon atoms which is optionally substituted by $C_1$-$C_6$-alkoxy, $C_1$-$C_6$-alkylthio,

$C_1$-$C_6$-alkylsulphonyl, halogen, cyano, hydroxyl, pyridyl, thienyl, pyrimidyl, piperidinyl, phenyl or an amino group, where the amino group carries two identical or different substituents from the series comprising $C_1$-$C_5$-alkyl, phenyl or benzyl,

$R^3$ represents $C_1$-$C_5$-alkyl, or represents cyano, hydroxymethyl or formyl, and

$R^4$ represents hydrogen, halogen, $C_1$-$C_5$-alkyl or trifluoromethyl,

in the form of their isomers, isomeric mixtures, racemates or optical antipodes, and their physiologically acceptable salts.

3. Process according to Claim 1 for the preparation of compounds of the formula I in which

$R^1$ represents hydrogen, fluorine, chlorine, bromine, nitro, phenyl, $C_1$-$C_4$-alkyl, $C_1$-$C_4$-alkoxy, trifluoromethyl or dimethylamino,

$R^2$ represents straight-chain or branched alkyl which has up to 6 carbon atoms and which may be substituted by methoxy, fluorine, chlorine, cyano, hydroxyl, pyridyl, phenyl or N-benzyl-N-methylamino,

$R^3$ represents methyl, and

$R^4$ represents hydrogen, fluorine or chlorine, in the form of their isomers, isomeric mixtures, racemates or optical antipodes, and their physiologically acceptable salts.

4. Process for the preparation of medicaments, characterized in that compounds prepared according to Claim 1 are optionally converted into a suitable administration form using conventional auxiliaries and excipients.

5. Process according to Claim 4 for the preparation of medicaments for coronary therapy, for influencing pathologically altered blood pressure, for treatment of heart insufficiency, for treatment of heart rhythm disturbances, for lowering blood sugar levels, for shrinking mucous membranes and for influencing the salt and liquid balance.

## Revendications

### Revendications pour les Etats Contractants : AT, BE, CH, DE, FR, GB, IT, LI, NL, SE

1. Benzylaminoaryl-dihydropyridinelactones de formule générale (I)

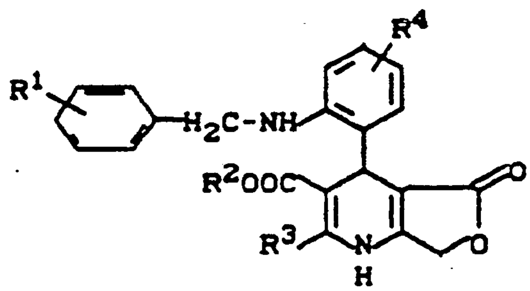

(I)

dans laquelle $R^1$ représente l'hydrogène, un halogène, un groupe cyano, nitro, aryle en $C_6$-$C_{12}$, alkyle en $C_1$-$C_8$, alcoxy en $C_1$-$C_8$, alkylthio en $C_1$-$C_6$, trifluorométhyle, trifluorométhoxy, trifluorométhylthio, difluorométhoxy, di-(alkyl en $C_1$-$C_5$)amino, (alcoxy en $C_1$-$C_6$)carbonyle ou alkylsulfonyle en $C_1$-$C_6$,

$R^2$ représente l'hydrogène ou un reste d'hydrocarbure saturé ou insaturé, à chaîne linéaire, ramifiée ou cyclique, avec jusqu'à 10 atomes de carbone, éventuellement substitué par un groupe alcoxy en $C_1$-$C_6$, un groupe alkylthio en $C_1$-$C_6$, un groupe alkylsulfonyle en $C_1$-$C_6$, un halogène, un groupe cyano, hydroxy, pyridyle, thiényle, pyrimidyle, pipéridinyle, phényle ou amino, le groupe amino portant deux substituants identiques ou différents de la série des groupes alkyle en $C_1$-$C_5$, phényle ou benzyle,

$R^3$ représente un groupe alkyle en $C_1$-$C_5$ ou un groupe cyano, hydroxyméthyle ou formyle et

$R^4$ représente l'hydrogène, un halogène, un groupe alkyle en $C_1$-$C_5$ ou un groupe trifluorométhyle, sous forme de leurs isomères, mélanges d'isomères, racémates, antipodes optiques, et leurs sels physiologiquement inoffensifs.

2. Composés selon la formule I de la revendication 1, dans laquelle .

$R^1$ représente l'hydrogène, le fluor, le chlore, le brome, l'iode, un groupe cyano, nitro, phényle, alkyle en $C_1$-$C_6$, alcoxy en $C_1$-$C_6$, alkylthio en $C_1$-$C_4$, trifluorométhyle, trifluorométhoxy, difluorométhoxy, di(alkyl en $C_1$-$C_3$)amino, (alcoxy en $C_1$-$C_4$)carbonyle ou alkylsulfonyle en $C_1$-$C_4$,

$R^2$ représente l'hydrogène ou un reste d'hydrocarbure à chaîne linéaire ou ramifiée avec jusqu'à 8 atomes

de carbone, qui peut être substitué par un groupe alcoxy en $C_1$-$C_4$, alkylthio en $C_1$-$C_4$, alkylsulfonyle en $C_1$-$C_4$, par le fluor, le chlore, le brome, l'iode, un groupe cyano, hydroxy, pyridyle, pyrimidyle, phényle ou par un groupe amino, le groupe amino portant deux substituants identiques ou différents de la série alkyle en $C_1$-$C_3$ ou benzyle,

$R_3$ représente un groupe alkyle en $C_1$-$C_3$ ou un groupe cyano et

$R_4$ représente l'hydrogène, le fluor, le chlore, le brome, un groupe alkyle en $C_1$-$C_3$ ou un groupe trifluorométhyle,

sous forme de leurs isomères, mélanges d'isomères, racémates, antipodes optiques, et leurs sels physiologiquement inoffensifs.

3. Composés selon la formule I de la revendication, dans laquelle

$R^1$ représente l'hydrogène, le fluor le chlore, le brome, un groupe nitro, phényle, alkyle en $C_1$-$C_4$, alcoxy en $C_1$-$C_4$, trifluorométhyle ou diméthylamino,

$R^2$ représente un groupe alkyle à chaîne linéaire ou ramifiée avec jusqu'à 6 atomes de carbone, pouvant être substitué par un groupe méthoxy, par le fluor, le chlore, un groupe cyano, hydroxy, pyridyle, phényle ou N-benzyl-N-méthylamino,

$R^3$ représente un groupe méthyle et

$R^4$ représente l'hydrogène, le fluor ou le chlore, sous forme de leurs isomères, mélanges d'isomères, racémates, antipodes optiques, et leurs sels physiologiquement inoffensifs.

4. Composés selon les revendications 1 à 3 pour lutter contre les maladies.

5. Médicaments contenant comme substance active un composé selon les revendications 1 à 3.

6. Procédé pour fabriquer les benzylaminoaryldihydropyridinelactones de formule générale (I)

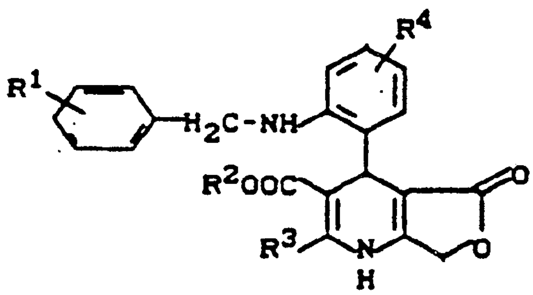

(I)

dans laquelle

$R^1$ représente l'hydrogène, un halogène, un groupe cyano, nitro, aryle en $C_6$-$C_{12}$, alkyle en $C_1$-$C_8$, alcoxy en $C_1$-$C_8$, alkylthio en $C_1$-$C_6$ trifluorométhyle trifluorométhoxy trifluorométhylthio, difluorométhoxy, di(alkyl en $C_1$-$C_5$)amino, (alcoxy en $C_1$-$C_6$)carbonyle ou alkylsulfonyle en $C_1$-$C_6$,

$R^2$ représente l'hydrogène ou un reste d'hydrocarbure saturé ou insaturé, à chaîne linéaire, ramifiée ou cyclique, avec jusqu'à 10 atomes de carbone, éventuellement substitué par un groupe alcoxy en $C_1$-$C_6$, un groupe alkylthio en $C_1$-$C_6$, un groupe alkylsulfonyle en $C_1$-$C_6$, un halogène, un groupe cyano, hydroxy, pyridyle, thiényle, pyrimidyle, pipéridinyle, phényle ou amino, le groupe amino portant deux substituants identiques ou différents de la série des groupes alkyle en $C_1$-$C_5$, phényle ou benzyle,

$R^3$ représente un groupe alkyle en $C_1$-$C_5$ ou un groupe cyano, hydroxyméthyle ou formyle et

$R^4$ représente l'hydrogène, un halogène, un groupe alkyle en $C_1$-$C_5$ ou un groupe trifluorométhyle, sous forme de leurs isomères, mélanges d'isomères, racémates, antipodes optiques, et leurs sels physiologiquement inoffensifs, caractérisé en ce qu'on fait réagir des composés aminés de formule générale (II) ci-dessous dans laquelle $R^2$, $R^3$ et $R^4$ ont les significations décrites ci-dessus

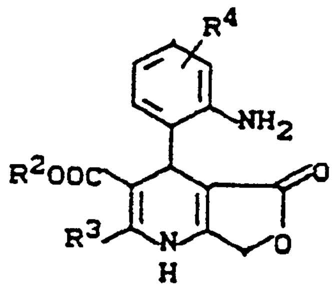

(II),

ou leurs sels, avec des halogénures de benzyle de formule générale (III)

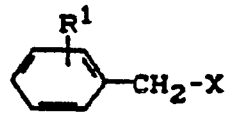

(III),

dans laquelle

$R^1$ possède la signification décrite ci-dessus et

X représente un halogène, de préférence le chlore ou le brome, éventuellement en présence de bases, éventuellement en présence d'un solvant inerte.

7. Procédé selon la revendication 6 pour la fabrication de composés de formule I, dans laquelle

$R^1$ représente l'hydrogène, le fluor, le chlore, le brome, l'iode, un groupe cyano, nitro, phényle, alkyle en $C_1$-$C_6$, alcoxy en $C_1$-$C_6$, alkylthio en $C_1$-$C_4$, trifluorométhyle, trifluorométhoxy, difluorométhoxy, di(alkyl en $C_1$-$C_3$)amino, (alcoxy en $C_1$-$C_4$)carbonyle ou alkylsulfonyle en $C_1$-$C_4$,

$R^2$ représente l'hydrogène ou un reste d'hydrocarbure saturé ou insaturé, à chaîne linéaire, ramifiée ou cyclique, avec jusqu'à 10 atomes de carbone, éventuellement substitué par un groupe alcoxy en $C_1$-$C_6$, un groupe alkylthio en $C_1$-$C_6$, un groupe alkylsulfonyle en $C_1$-$C_6$, un halogène, un groupe cyano, hydroxy, pyridyle, thiényle, pyrimidyle, pipéridinyle, phényle ou amino, le groupe amino portant deux substituants identiques ou différents de la série des groupes alkyle en $C_1$-$C_5$, phényle ou benzyle,

$R^3$ représente un groupe alkyle en $C_1$-$C_5$ ou un groupe cyano, hydroxyméthyle ou formyle et

$R^4$ représente l'hydrogène, un halogène, un groupe alkyle en $C_1$-$C_5$ ou un groupe trifluorométhyle, sous forme de leurs isomères, mélanges d'isomères, racémates, antipodes optiques, et leurs sels physiologiquement inoffensifs.

8. Procédé selon la revendication 6 pour la fabrication de composés de formule I dans laquelle

$R^1$ représente l'hydrogène, le fluor, le chlore, le brome, un groupe nitro, phényle, alkyle en $C_1$-$C_4$, alcoxy en $C_1$-$C_4$, trifluorométhyle ou diméthylamino,

$R^2$ représente un groupe alkyle à chaîne linéaire ou ramifiée avec jusqu'à 6 atomes de carbone, pouvant être substitué par un groupe méthoxy, par le fluor, le chlore, un groupe cyano, hydroxy, pyridyle, phényle ou N-benzyl-N-méthylamino,

$R^3$ représente un groupe méthyle et

$R^4$ représente l'hydrogène, le fluor ou le chlore, sous forme de leurs isomères, mélanges d'isomères, racémates, antipodes optiques, et leurs sels physiologiquement inoffensifs.

9. Procédé pour la fabrication de médicaments selon la revendication 5, caractérisé en ce qu'on mélange de manière usuelle les substances actives selon les revendications 1 à 3 avec des adjuvants et/ou des excipients usuels.

10. Utilisation des composés selon les revendications 1 à 3 pour la fabrication de médicaments destinés à la thérapeutique coronaire pour influencer une pression sanguine pathologiquement modifiée, pour traiter l'insuffisance cardiaque, pour traiter l'arythmie cardiaque, pour abaisser la glycémie, pour décongestionner les muqueuses et pour influencer le bilan des sels et des liquides.

**Revendications pour les Etats Contractants ES, GR**

1. Procédé pour la fabrication de benzylaminoaryldihydropyridinelactones de formule générale (I)

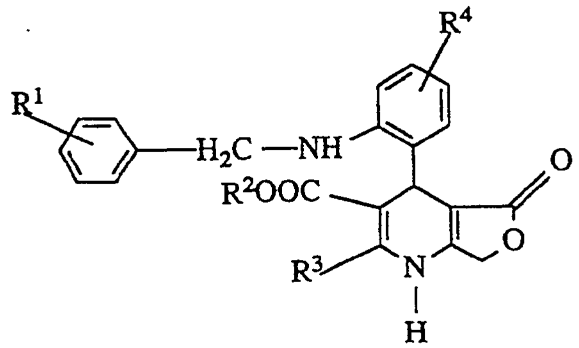

(I)

dans laquelle

$R^1$ représente l'hydrogène, un halogène un groupe cyano, nitro, aryle en $C_6$-$C_{12}$, alkyle en $C_1$-$C_8$, alcoxy en $C_1$-$C_8$, alkylthio en $C_1$-$C_6$, trifluorométhyle, trifluorométhoxy, trifluorométhylthio, difluorométhoxy, di(alkyl en $C_1$-$C_5$)amino, (alcoxy en $C_1$-$C_6$)carbonyle ou alkylsulfonyle en $C_1$-$C_6$,

$R^2$ représente l'hydrogène ou un reste d'hydrocarbure saturé ou insaturé, à chaîne linéaire, ramifiée ou cyclique, avec jusqu'à 10 atomes de carbone, éventuellement substitué par un groupe alcoxy en $C_1$-$C_6$, un groupe alkylthio en $C_1$-$C_6$, un groupe alkylsulfonyle en $C_1$-$C_6$, un halogène, un groupe cyano, hydroxy, pyridyle, thiényle, pyrimidyle, pipéridinyle, phényle ou amino, le groupe amino portant deux substituants identiques ou différents de la série des groupes alkyle en $C_1$-$C_5$, phényle ou benzyle,

$R^3$ représente un groupe alkyle en $C_1$-$C_5$ ou un groupe cyano, hydroxyméthyle ou formyle et

$R^4$ représente l'hydrogène, un halogène, un groupe alkyle en $C_1$-$C_5$ ou un groupe trifluorométhyle, sous forme de leurs isomères, mélanges d'isomères, racémates, antipodes optiques, et leurs sels physiologiquement inoffensifs, caractérisé en ce qu'on fait réagir des composés aminés de formule générale (II) ci-dessous dans laquelle $R^2$, $R^3$ et $R^4$ ont les significations décrites ci-dessus

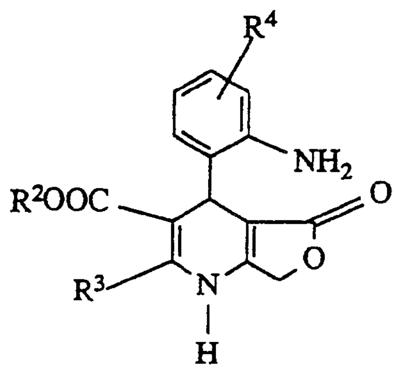

(II)

ou leurs sels, avec des halogénures de benzyle de formule générale (III)

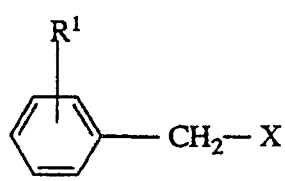

(III)

dans laquelle

$R^1$ possède la signification décrite ci-dessus et

X représente un halogène, de préférence le chlore ou le brome, éventuellement en présence de bases, éventuellement en présence d'un solvant inerte.

2. Procédé selon la revendication 6 pour la fabrication de composés de formule I, dans laquelle $R^1$ représente l'hydrogène, le fluor, le chlore, le brome, l'iode, un groupe cyano, nitro, phényle, alkyle en $C_1$-$C_6$, alcoxy en $C_1$-$C_8$, alkylthio en $C_1$-$C_4$, trifluorométhyle, trifluorométhoxy, difluorométhoxy, di(alkyl en $C_1$-$C_3$)amino, (alcoxy en $C_1$-$C_4$) carbonyle ou alkylsulfonyle en $C_1$-$C_4$,

$R^2$ représente l'hydrogène ou un reste d'hydrocarbure saturé ou insaturé, à chaîne linéaire, ramifiée ou cyclique, avec jusqu'à 10 atomes de carbone, éventuellement substitué par un groupe alcoxy en $C_1$-$C_6$, un groupe alkylthio en $C_1$-$C_6$, un groupe alkylsulfonyle en $C_1$-$C_6$, un halogène, un groupe cyano, hydroxy, pyridyle, thiényle, pyrimidyle, pipéridinyle, phényle ou amino, le groupe amino portant deux substituants identiques ou différents de la série des groupes alkyle en $C_1$-$C_5$, phényle ou benzyle,

$R^3$ représente un groupe alkyle en $C_1$-$C_5$ ou un groupe cyano, hydroxyméthyle ou formyle et

$R^4$ représente l'hydrogène, un halogène, un groupe alkyle en $C_1$-$C_5$ ou un groupe trifluorométhyle, sous forme de leurs isomères, mélanges d'isomères, racémates, antipodes optiques, et leurs sels physiologiquement inoffensifs.

3. Procédé selon la revendication 6 pour la fabrication de composés de formule I dans laquelle

$R^1$ représente l'hydrogène, le fluor, le chlore, le brome, un groupe nitro, phényle, alkyle en $C_1$-$C_4$, alcoxy en $C_1$-$C_4$, trifluorométhyle ou diméthylamino,

$R^2$ représente un groupe alkyle à chaîne linéaire ou ramifiée avec jusqu'à 6 atomes de carbone, pouvant être substitué par un groupe méthoxy, par le fluor, le chlore, un groupe cyano, hydroxy, pyridyle, phényle ou N-benzyl-N-méthylamino,

$R^3$ représente un groupe méthyle et

$R^4$ représente l'hydrogène, le fluor ou le chlore, sous forme de leurs isomères, mélanges d'isomères, racémates, antipodes optiques, et leurs sels physiologiquement inoffensifs.

4. Procédé pour la fabrication de médicaments caractérisé en ce qu'on incorpore les composés fabriqués selon la revendication 1 dans une forme appropriée d'administration, éventuellement avec des adjuvants et excipients usuels.

5. Procédé selon la revendication 4 pour la fabrication de médicaments destinés à la thérapeutique coronaire pour influencer une pression sanguine pathologiquement modifiée, pour traiter l'insuffisance cardiaque, pour traiter l'arythmie cardiaque, pour abaisser la glycémie, pour décongestionner les muqueuses et pour influencer le bilan des sels et des liquides.